# EUROPÄISCHE PATENTSCHRIFT

(11) **EP 4 578 431 B1**
(45) Veröffentlichungstag und Bekanntmachung des Hinweises auf die Patenterteilung: **19.08.2026**
(21) Anmeldenummer: 23220303.4
(22) Anmeldetag: 27.12.2023
(51) Int. Cl.: A61F 13/00, A61F 13/0206

(54) **DRUCKSTABILES, ZUSCHNEIDBARES WUNDVERSORGUNGSPRODUKT FÜR NEKROTISCHE ULCERA**
PRESSURE-STABLE, TRIMABLE WOUND CARE PRODUCT FOR NECROTIC ULCERS
PRODUIT D'ENTRETIEN DE PLAIE, STABLE À LA PRESSION, POUVANT ÊTRE COUPÉ POUR ULCÉREUX NÉCROSIQUES

(43) Veröffentlichungstag der Anmeldung: 02.07.2025
(73) Patentinhaber: Paul Hartmann AG, 89522 Heidenheim (DE)
(72) Erfinder: MARZ, Jacqueline, 8212 Neuhausen (CH); ROTHMAIER, Markus, 8212 Neuhausen (CH)
(74) Vertreter: Paul Hartmann AG Patents & Licensing

(56) Entgegenhaltungen:
- EP-B1- 3 315 145
- AU-A1- 2012 236 039
- DE-A1- 102013 105 063
- DE-U1- 202013 104 893

## Beschreibung

### Technisches Gebiet der Erfindung

Die Erfindung betrifft ein textiles, mehrschichtiges Wundversorgungsprodukt, welches geeignet ist zur Behandlung unterschiedlicher Wunden, insbesondere Ulcera und besonders Ulcera an der Fußsohle. Das Wundversorgungsprodukt ist durch seinen speziellen Aufbau in der Lage ein physikalisch-autolytisches Debridement durchzuführen, dadurch Nekrosen aus der Wunde schonend zu entfernen und hierdurch den Wundverschluss einzuleiten.

### Hintergrund der Erfindung

Wundauflagen für die feuchte Wundversorgung sind aus dem Stand der Technik grundsätzlich bekannt.

Eine Variante wird in der WO 2011/141454 A1 beschrieben. Es handelt sich hierbei um eine wundkissenartige oder kompressenartige Wundauflage, die auf eine Wunde aufbringbar ist oder auch zum Austamponieren tiefer Wunden verwendet werden kann. Bestandteil dieses Produkts ist ein Saug-/Spülkörper, welcher über eine umlaufende Schweißnaht verfügt und herstellerseitig mit einer salzhaltigen wässrigen Lösung beaufschlagt ist, wodurch das enthaltene superabsorbierende Material aufquillt und in einen gelartigen Zustand übergeht. Dies verleiht dem Saug-/Spülkörper eine duale Funktion bei Wunden mit starker Exsudation: Wundsekrete werden einschließlich der darin enthaltenen Bestandteile wie Keime durch den Saug-/Spülkörper aktiv aufgenommen und darin gehalten, wobei der Saug-/Spülkörper im Austausch die salzhaltige wässrige Lösung an die Wunde abgibt und somit ein feuchtes Wundmilieu schafft oder unterstützt. Hierdurch wird die Wundreinigung und eine positive Wundkonditionierung unterstützt und somit die Heilung positiv beeinflusst. Dies wird als interaktive Nasstherapie bezeichnet, die insbesondere bei schlecht heilenden Wunden, bei klinisch manifest infizierten Wunden oder bei chronischen Wunden mit unterschiedlicher Genese, wie diabetisches Gangrän, Dekubitalulcus oder Ulcus cruris bevorzugt Anwendung findet. Wundauflagen dieses Typs haben jedoch den Nachteil, dass sie (nach Herstellung) nicht dafür vorgesehen sind, an die Größe der Wunde angepasst, z.B. zugeschnitten, zu werden. Grund hierfür ist, dass beim Zuschneiden die äußere Barriere inklusive der Schweißnaht durchtrennt wird und das Gel anschließend herausquillt. Ein Zuschneiden im trockenen Zustand mit späterer Flüssigkeitszugabe ist ebenfalls nicht möglich, da nach dem Schneiden die Superabsorberpartikel aus dem Inneren herausfallen und schlimmstenfalls in der Wunde verbleiben. Auch ohne ein Zuschneiden des Produktes kann es passieren, dass unter starkem Druck die Schweißnaht reißt und das Gel bzw. die Partikel aus superabsorbierendem Polymer (SAP) austreten. Deswegen ist ein solches Produkt aus dem Stand der Technik nicht für die Behandlung von Ulcera an der Fußsohle, wie sie häufig bei Diabetikern auftreten, geeignet bzw. kann nur genutzt werden, wenn der Patient das Auftreten vermeidet, beispielsweise durch eine liegende Position oder eine Hochlagerung des betroffenen Beines. Andernfalls droht die Wundauflage zu platzen, sobald der Patient sein Gewicht auf den zu behandelnden Fuß verlagert. Die Behandlung im Liegen oder der Einsatz von Krücken sind belastend für den Patienten. Ausdauerndes Liegen erhöht das Thromboserisiko und Krücken empfinden vor allem ältere Patienten als übermäßig belastend.

Das in Bezug auf die WO 2011/141454 A1 Dargestellte gilt in nahezu identischer Weise für die Wundauflage der WO 2016/156619 A1. Auch diese ist für die feuchte Wundversorgung vorgesehen und wird in Form eines Flächenmaterials bereitgestellt, das aus mehreren Schichten besteht, die lediglich am Rand durch eine Schweißnaht miteinander verbunden sind. Die Schichten können sich ansonsten z.B. bei Körperbewegungen gegeneinander verschieben, so dass eine flexible Anordnung entsteht. Auch diese Wundauflage ist nicht zuschneidbar und nicht für die Anwendung an der Fußsohle geeignet.

Zwar können die oben genannten Produkte für die Versorgung von Ulcera, z.B. in Form eines offenen Beines, genutzt werden, allerdings entsteht auch hier häufig ein Problem: Da die Produkte nicht an die Größe der Wunde zugeschnitten werden können, überragen sie die Wunde zwangsläufig und da sie in feuchter Form appliziert werden, wird die gesunde Haut, welche die Wunde umgibt, mit zunehmender Anwendungsdauer aufquellen. In der Fachsprache wird dieser unerwünschte Effekt als Mazeration bezeichnet und kann im ungünstigsten Fall zu einer Vergrößerung der Wunde führen.

Weiterer Stand der Technik ist aus DE202013104893U1, DE102013105063, AU2012236039 und EP3315145 bekannt.

Folglich besteht ein Bedarf für eine Wundauflage, die für die Behandlung unterschiedlicher Wunden, insbesondere von Ulcera, und zwar auch an der Fußsohle, geeignet ist, zugeschnitten werden kann, Nekrosen entfernt und ein feuchtes Wundmilieu schafft, so dass der Wundverschluss gefördert wird, und zwar ohne, dass der Patient während der Behandlung in seiner Bewegungsfreiheit eingeschränkt wird. Ferner soll eine Mazeration der Wundränder vermieden werden.

### Zusammenfassung der Erfindung

Die vorangestellte Aufgabe wird gelöst durch die Bereitstellung eines Wundversorgungsproduktes umfassend die folgenden Bestandteile:
a) eine proximal gelegene flüssigkeitsdurchlässige Schicht mit einem Vliesstoff,
b) eine distal gelegene Schutzschicht mit einem Vliesstoff,
c) eine zwischen der proximal gelegenen flüssigkeitsdurchlässigen Schicht und der distal gelegenen Schutzschicht gelegene Quellschicht in Form eines Vliesstoffes mit superabsorbierenden, polymerhaltigen Fasern, wobei die superabsorbierenden Fasern mit mindestens einer anderen Faserart in einem Filz vorliegen,
d) eine wässrige Salzlösung, die in der Quellschicht eingelagert ist, im eingelagerten Zustand einen pH < 7,0 hat und die während einer Wundbehandlung an eine Wunde abgegeben werden kann,
wobei die proximal gelegene flüssigkeitsdurchlässige Schicht, die Quellschicht und die distal gelegene Schutzschicht einen identischen Zuschnitt haben und ohne Überstand vollflächig aneinanderliegen, und wobei das Wundversorgungsprodukt nahtlos ausgestaltet ist.

Alternativ kann das Wundversorgungsprodukt aus den oben genannten Bestandteilen bestehen.

Durch den beschriebenen Aufbau kann die erfindungsgemäße Wundauflage auf die Größe der Wunde zugeschnitten werden und falls eine Anwendung an der Fußsohle vorgesehen ist, bleibt der Patient während der Behandlung mobil. Weiterhin wird ein mögliches Überstehen der Wundauflage über die Fußsohle hinaus vermieden, so dass z.B. das Tragen von Schuhen weiterhin möglich ist. Bei einer Verlagerung des Körpergewichts auf die Wundauflage wird die Wunde verstärkt über die Salzlösung gespült und bei der nachfolgenden Entlastung (Belastung des anderen Beins) wird die Lösung größtenteils wieder von der Wundauflage aufgenommen. Hierdurch erfolgt ein repetitives physikalisch-autolytisches Debridement, welches Zelltrümmer, Eiter und vor allem nekrotische Beläge entfernen kann. Gleichzeitig werden - im Falle einer Wundinfektion - Erreger aus der Wunde gespült und von der Wundauflage aufgenommen, wo sie zurückgehalten werden. Zusammen mit dem feuchten Wundmilieu werden optimale Bedingungen geschaffen, um insbesondere auch chronische Wunden zum Abheilen anzuregen und die Lebensqualität der betroffenen Patienten zu steigern.

### Detaillierte Beschreibung der Erfindung

Der Ausdruck "zuschneidbar" meint, dass ein Produkt oder Material mit einem handelsüblichen mechanischen Werkzeug wie beispielsweise einer Schere gezielt vor der Anwendung auf eine vom Anwender vorgesehene Größe verkleinert werden kann, ohne dass das Produkt oder Material an Funktion einbüßt, und ohne dass ein unerwünschter (teilweiser oder vollständiger) Zerfall des Produktes oder Materials einsetzt.

Der Begriff "medizinisch akzeptables Material" im Sinne der Erfindung ist eine ungiftige, fusselfreie und beständige Substanz, die sich unter Normalbedingungen weder in polaren noch in unpolaren Substanzen zersetzt und weder von den Absonderungen tierischer noch bakterieller Zellen in nennenswertem Ausmaß abgebaut werden kann.

Der Ausdruck "atraumatisch" meint, dass ein Wundversorgungsprodukt sich nicht mit der Wunde fest verbindet, also beispielsweise nicht in der Wunde eintrocknet oder mit dieser verwächst und dass eine schmerzfreie Entfernung des Produktes ohne Störung des Heilungsprozesses möglich ist.

Unter dem Ausdruck "konfiguriert zum Auflegen auf eine Wunde" wird verstanden, dass das Wundversorgungsprodukt zum Auflegen auf eine Wunde zu einem therapeutischen Zweck im Sinne einer Wundbehandlung vorgesehen ist.

Unter "Ringerlösung" wird eine wässrige Lösung mit Natriumchlorid, Kaliumchlorid und Calciumchlorid (insbesondere 8,6 g NaCl, 0,3 g KCI und 0,33 g CaCl2 je Liter Wasser) verstanden, welche im Wesentlichen isotonisch ist (Osmolarität von etwa 308 mOsm/l). Ringerlösung wird in der Regel vor dem Einsatz sterilisiert.

"Proximal" meint im Sinne der vorliegenden Erfindung, dass ein Material oder ein Stoff eine Position innerhalb eines Wundversorgungsproduktes einnimmt, so dass er innerhalb dieses Produktes während der Anwendung in der Wundversorgung zur Wunde hin angeordnet ist. Dies ist beispielsweise bei einer Wundkontaktschicht der Fall.

"Distal" meint im Sinne der vorliegenden Erfindung, dass ein Material oder ein Stoff eine Position innerhalb eines Wundversorgungsproduktes einnimmt, so dass er innerhalb dieses Produktes während der Anwendung in der Wundversorgung von der Wunde weg angeordnet ist, wie es beispielsweise bei einem Backing (abschließende Stützschicht) der Fall ist.

"SAF" ist eine Abkürzung, die für superabsorbierende Fasern steht. Derartige Fasern sind in der Lage ein Vielfaches ihres Eigengewichts an polaren Flüssigkeiten zu absorbieren und können zusammen mit anderen Fasern zu einem stabilen Faserverbund verarbeitet werden.

"Zusatzfaser" meint eine Faser, die sich von superabsorbierenden Fasern (SAF) unterscheidet und somit selbst keine SAF ist.

"Moisture Vapor Transmission Rate" oder "MVTR" meint einen Messwert, der die Durchlässigkeit von Wasserdampf durch ein bestimmtes Material in g/m2/24h, angibt. Die MVTR kann mithilfe des Standards DIN EN 13726-2 (Juni 2002) bestimmt werden.

Das erfindungsgemäße Wundversorgungsprodukt besteht aus mindestens drei Schichten: einer proximal gelegenen flüssigkeitsdurchlässigen Schicht, einer Quellschicht und einer distal gelegenen Schutzschicht. Alle drei dieser Schichten haben einen identischen Zuschnitt, so dass sie ohne Überstand vollflächig aneinanderliegen. Geringe produktionsbedingte Abweichungen (Toleranzen) sind jedoch möglich, ohne dass die Funktion eingeschränkt wird. Dabei können eine oder zwei Schichten bis zu 5 mm über eine andere Schicht bzw. Schichten hinausragen.

Die proximal gelegene flüssigkeitsdurchlässige Schicht kann als Wundkontaktschicht fungieren. Alternativ ist es möglich das Wundversorgungsprodukt mit einer zusätzlichen silikonhaltigen Schicht auszustatten, welche dann die Rolle der Wundkontaktschicht übernimmt und hervorragende atraumatische Eigenschaften bietet. Das entsprechende Vorgehen wird an anderer Stelle im Detail erläutert.

Die distal gelegene Schutzschicht überdeckt die Quellschicht in distaler Richtung, schützt diese vor äußeren Einflüssen und erleichtert die Handhabung des Wundversorgungsproduktes durch eine Verbesserung der haptischen Eigenschaften. Die Wirkung der Schutzschicht kann durch die zusätzliche Applikation eines Backing weiter gesteigert werden, was an anderer Stelle im Detail erläutert wird.

Die Druckresistenz wird vermittelt durch den Aufbau des Produktes, wobei die Zusammensetzung der Quellschicht umfassend einen Filz aus SAF und Zusatzfasern sowie der dadurch ermöglichte Verzicht auf eine Schweißnaht entscheidend sind. Nahtlos bedeutet insbesondere, dass das Produkt über keine Schweißnaht verfügt. Auf diese Wiese können die erforderlichen Druckbelastbarkeitswerte erreicht werden. Demgemäß reagiert das Wundversorgungsprodukt unter Einwirkung eines mechanischen Drucks lediglich durch elastische und somit reversible Verformung. So kann bevorzugt vorgesehen sein, dass das Wundversorgungsprodukt eine Druckresistenz gegenüber einem Druck von mindestens 8 kg / 100 cm2 , bevorzugt 20 kg / 100 cm2, noch besser mindestens 50 kg / 100 cm2 und am besten 120 kg / 100 cm2 aufweist. Der Druckresistenzwert ist zu verstehen in Bezug auf eine Kraft, die sich über die gesamte (distale bzw. proximale) Fläche des Wundversorgungsproduktes verteilt (vollflächige Druckresistenz).

Die Druckresistenz kann mithilfe des folgenden Verfahrens getestet werden:
1) Bereitstellen eines mit wässriger Salzlösung behandelten Wundversorgungsproduktes, dessen Sterilisation innerhalb der letzten 14 Tagen stattgefunden hat.
2) Einlegen des (unverpackten) Produktes in einen handelsüblichen, transparenten ZIP-Beutel
3) Aufbau der zu testenden Gewichtskraft innerhalb von einer Sekunde
4) Halten der Gewichtskraft für eine Sekunde
5) Abbau der Gewichtskraft innerhalb von zwei Sekunden
6) Wiederholung der Schritte 3 bis 5, so dass die Gewichtskraft insgesamt zehn Mal appliziert wird
7) Entnahme des Produktes aus dem Beutel
8) Visuelle Inspektion sowohl des Produktes als auch des Beutelinneren auf abgetrennte Fasern oder ausgetretenes Gel
Wenn weder am Produkt selbst noch im Beutelinneren abgetrennte Fasern oder ausgetretenes Gel erkennbar sind, gilt der Test für die entsprechende Gewichtskraft als bestanden.

Bevorzugt ist das Wundversorgungsprodukt derlei gestaltet, dass es aufgrund seiner elastischen Eigenschaften und der Druckbelastbarkeit um 90°, noch besser um 180° entlang einer tatsächlichen oder (bei unsymmetrischem Aufbau) gedachten Mittellinie umgeschlagen bzw. umgefaltet werden kann, und zwar ohne, dass eine plastische Verformung stattfindet.

Die proximal gelegene flüssigkeitsdurchlässige Schicht und die Schutzschicht können neben dem genannten Vliesstoff auch andere Bestandteile wie z.B. andere Faseranordnungen umfassen, solange diese nach einem Zuschneidevorgang nicht desintegrieren. Bevorzugt bestehen die flüssigkeitsdurchlässige Schicht und die Schutzschicht vollständig aus Vliesstoff, da Vliesstoffe besonders resistent gegen Zerfaserungen sind, und zwar auch nach einem Zuschneiden des Materials.

Die Quellschicht enthält superabsorbierende Fasern (SAF)), insbesondere superabsorbierende Fasern, welche ein superabsorbierendes Polymer enthalten. Das superabsorbierendes Polymer umfasst bevorzugt ein acrylathaltiges Polymer oder ein Acrylat-Co-Polymer. Die SAF können einen Faserdurchmesser von beispielsweise 50 bis 500 µm haben. Bevorzugt wird ein Faserdurchmesser von 100 bis 200 µm, da sich bei diesem Durchmesser ein ideales Wasser-zu-Faser-Verhältnis ausbilden kann. Hierdurch kann eine größere Menge, beispielsweise 15 ml, einer wässrigen Salzlösung aus der Quellschicht an die Wunde abgegeben, aber auch wieder aufgenommen werden. Dieser Mechanismus basiert zum einen auf chemisch-physikalischen Prozessen wie der Diffusion, bei welcher saubere Salzlösung in Richtung Wunde wandert und ausgespültes Wundexsudat in Richtung Quellschicht wandert. Zum anderen wird die Salzlösung unter mechanischem Druck teilweise herausgepresst und beim Aufheben der Druckkraft wieder von der Quellschicht aufgenommen. Möglichkeiten die SAF bereitzustellen und anzupassen werden an anderer Stelle näher erläutert.

Bevorzugt ist das Wundversorgungsprodukt flächig, hat also eine flache distal gelegene Oberseite und eine ebenfalls flache proximal gelegene Unterseite, so dass es hervorragend zur Wundabdeckung geeignet ist und bündig mit den Wundrändern abschließt. Flach meint, dass die jeweilige Schicht keine wesentlichen Erhebungen oder Vertiefungen aufweist. Als Oberseite kann die bereits erwähnte Schutzschicht dienen. Als Unterseite kann die ebenfalls bereits erwähnte proximal gelegene flüssigkeitsdurchlässige Schicht dienen. Hierdurch lässt sich das Produkt gut auf der Wunde drapieren. Die flache Unterseite unterstützt die atraumatischen Eigenschaften und vermindert die Wundreizung.

Das Wundversorgungsprodukt ist darüber hinaus bevorzugt rund oder ellipsenförmig (in der Aufsicht). Dies hat im Vergleich zu rechteckigen Formen den Vorteil, dass das Fabrikat bereits im ungeschnittenen Zustand der Form der meisten chronischen Wunden ähnelt, welche (im Gegensatz zu z.B. Schnittwunden) in dem meisten Fällen eine kreisähnliche Erscheinung haben. Zudem hat sich gezeigt, dass runde oder ellipsenförmige Formen besser und dauerhafter an der Fußsohle fixiert werden können und den Träger weniger stören als andere Formen wie z.B. rechteckige Ausgestaltungen mit abgerundeten Ecken, wie sie häufig bei Schürfwunden zum Einsatz kommen. Zudem haben rechteckige Ausgestaltungen den Nachteil, dass sie sich bei fortwährender mechanischer Beanspruchung - wie z.B. beim Gehen - im Bereich der Ecken schnell von der Haut abzulösen beginnen. Dabei zeigen die Ecken eine Tendenz sich aufzurollen, was zu einer ungewollten Erhebung führt und im Bereich der Fußsohle zu Druckschmerzen führen kann.

Zum Auflegen auf eine Wunde oder zum Abdecken einer Wunde wird die Schutzschicht in distaler Richtung bevorzugt mittels einer zusätzlichen Backingschicht überlagert, welche der Verdunstung der Salzlösung entgegenwirkt, so dass die feuchte Wundbehandlung über einen Zeitraum von mehreren Tagen durchgehend und ohne Verbandswechsel stattfinden kann. Die Backingschicht bzw. das Backing vermittelt zudem stützende Eigenschaften, die das faltenlose Anbringen oder Ankleben des Wundversorgungsproduktes vereinfachen. Vorteilhaft wirkt sich hierbei aus, dass die SAF in der Quellschicht hervorragende Wasserspeichereigenschaften haben. Das Filzmaterial der Quellschicht kann darüber hinaus die aus der Wunde ausgeschwemmten Stoffe und Schadorganismen (z.B. Bakterien) aufnehmen und zurückhalten, bis diese beim nächsten Verbandswechsel entsorgt werden.

Das erfindungsgemäße Wundversorgungsprodukt enthält eine Quellschicht in Form eines Vliesstoffes mit superabsorbierenden, polymerhaltigen Fasern, wobei die superabsorbierenden Fasern mit mindestens einer anderen Faserart (Zusatzfaser) in einem Filz als Faserverbund vorliegen. Sinn und Zweck dieser weiteren Fasern ist es der Quellschicht ausreichende Reiß- und Zugfestigkeit sowie Formstabilität im feuchten Zustand zu verleihen. Da die SAF mit der Zusatzfaser als Filz vorliegen, bilden diese einen stabilen Faserverbund.

In Bezug auf die Quellschicht kann es sich bei der Zusatzfaser ("anderen Faser") um eine Faserart synthetischen, natürlichen oder halb-synthetischen Ursprungs handeln, wobei SAF explizit ausgenommen sind. Beispiele für geeignete Fasern synthetischen Ursprungs sind Polyolefin-basierte Fasern wie Polyester und Polyamid. Innerhalb der Gruppe der Polyester ist Polyethylenterephthalat besonders geeignet. Beispiele für geeignete Fasern natürlichen Ursprungs sind wie Baumwolle und Flachs. Ein Beispiel für Fasern halb-synthetischen Ursprungs ist Lyocell. Natürliche und halb-synthetische Fasern bilden gemeinsam die Gruppe der zellulosehaltigen Fasern, sofern sie Zelluloseanteile enthalten.

Die Zusatzfaser liegt zusammen mit der SAF in einem Filz, bevorzugt einem Nadelfilz, vor. Das heißt, dass die beiden Fasertypen gemeinsam verfilzt sind. Der Filz kann als Nassfilz oder als Trockenfilz hergestellt werden. Die Herstellung als Trockenfilz ist bevorzugt, da so eine Rücktrocknung der SAF vermieden werden kann. Der Trockenfilz kann als Nadelfilz, Klebefilz oder thermisch verfestigtes Schmelzfilz bereitgestellt werden. Bei einem Nadelfilz werden die Zusatzfasern und die SAF maschinell miteinander zu einem Filz vernadelt. Dabei kann die andere Faserart (Zusatzfaser) mit den superabsorbierenden, polymerhaltigen Fasern derart vernadelt sein, dass sie in einem Nadelfilz vorliegt. Bei einem Klebefilz werden die beiden Faserarten durch Beimengen eines Klebstoffs (hauptsächlich durch chemische Wechselwirkung) miteinander verbunden. Der verwendete Klebstoff sollte nach der Aushärtung nicht wasserlöslich sein, da es ansonsten durch Einwirkung der wässrigen Salzlösung zu einem Zerfall kommen könnte. Bei einem Schmelzfilz wird die Zusatzfaser aus dem Bereich der Schmelzfasern gewählt und mit den SAF (welche inhärente Schmelzfasereigenschaften besitzen) durch Einsatz thermischer Energie miteinander verbunden. Bevorzugt liegen die Fasern der Quellschicht als Nadelfilz bzw. im Nadelfilz vor. Eine Quellschicht in Form eines Nadelfilzes bietet den Vorteil, dass sie kompatibel zu sämtlichen Sterilisationsmethoden ist, da sie hervorragende Beständigkeit gegenüber Hitze, Druck, Wasserdampf, Strahlung und Ethylenoxid zeigt. Darüber hinaus lässt die Nadelfilzform große Freiheit bei der Auswahl der Fasern und weist eine sehr lange Haltbarkeitsdauer selbst bei langer Lagerung auf.

Die derart aufgebaute Quellschicht kann problemlos geschnitten werden, ohne dass sich die SAF und die Zusatzfaser voneinander lösen, und ohne dass Superabsorberpartikel, wie sie häufig in absorbierenden Wundauflagen verwendet werden, aus der Quellschicht im Schnittbereich herausfallen. Des Weiteren wird eine dauerhafte Befestigung an den anderen Schichten des Wundversorgungsproduktes ermöglicht. So ist es beispielsweise möglich die Quellschicht auf ihrer distalen und proximalen Seite mit anderen Materialien zu verkleben. Beispiele, wie derartige Laminate erhältlich sind, werden in den Ausführungsbeispielen beschrieben.

Das erfindungsgemäße Wundversorgungsprodukt ist hervorragend geeignet für die feuchte Wundbehandlung. Dabei meint "geeignet für die feuchte Wundbehandlung" bzw. "zur feuchten Wundbehandlung", dass das Produkt ohne weitere Vorbereitung oder Modifikation derart eingesetzt werden kann, dass die feuchte Wundbehandlung unmittelbar mit der Applikation des Produktes einsetzt. Damit einhergehend kann das Wundversorgungsprodukt zwischen Herstellung und Einsatz im feuchten Zustand gelagert werden, ohne dass es zu einer Beeinträchtigung der strukturellen Integrität kommt.

Die in der Quellschicht enthaltene Salzlösung spült die Wunde, löst hierdurch Belege wie z.B. Fibrin, unterstützt die autokatalytische Zersetzung von Nekrosen und entfernt Mikroorganismen. Überschüssige Matrix Metalloproteinasen werden aus der Wunde gespült und stagnierte Heilungsprozesse neu angestoßen. Das feuchte Milieu beschleunigt zudem generell den Wundverschluss.

Sofern das Produkt an der Fußsohle angebracht ist, wird durch die Bewegung der venöse Abfluss des Blutes angeregt, was vor allem bei chronischen Wunden, die auf Venenstau zurückzuführen sind (z.B. Ulcus cruris auch bekannt als "offenes Bein") von großer Bedeutung ist. Da die Form des Produktes durch das Zuschneiden an die Form der Wunde angepasst werden kann, wird ein Überragen der Wunde und damit eine Mazeration der die Wunde umgebenden Haut trotz der feuchten Wundversorgung vermieden. Zudem lässt sich das Produkt auf diese Weise deutlich komfortabler unter eine Kompressionsbandage oder einem Kompressionsstrumpf tragen, wie sie bei Druckgeschwüren zum Einsatz kommen, um die pathologische venöse Stauung zu beheben.

Das Wundversorgungsprodukt gemäß der vorliegenden Erfindung ist hervorragend zur Behandlung, insbesondere zur feuchten Wundbehandlung, von schwer oder nicht heilenden und chronischen Wunden geeignet. Hierunter fallen insbesondere die folgenden Wundarten: diabetische Fußläsionen, Ulcus cruris venosum, Ulcus cruris arteriosum, Ulcus cruris mixtum und Dekubitus. Diese Wunden zeichnen sich häufig durch die Anwesenheit von Belägen (z.B. Fibrinbelägen) und Nekrosen aus. Weiterhin scheint der gehemmte Heilungsprozess mit einem Ungleichgewicht der notwendigen Botenstoffe, Enzyme (z.B. Matrix-Metalloproteasen) und molekularen Inhibitoren in der Wunde einherzugehen. Durch die Spülung der Wunde im Rahmen der feuchten Wundbehandlung wird das zuvor vorherrschende chemische Ungleichgewicht beseitigt und es kann sich ein für die Wundheilung förderliches Milieu einstellen. Die zuvor gehemmte Heilung wird auf diese Weise wieder in Gang gesetzt. Wie bereits erwähnt, lässt sich das erfindungsgemäße Wundversorgungsprodukt in den allermeisten Fällen mit einer Kompressionstherapie kombinieren, um den venösen Rückstau als ursächliches Ulcus-auslösendes Problem, zu beseitigen.

Gemäß einer Ausführungsform der Erfindung ist vorgesehen, dass das Wundversorgungsprodukt eine distal zur Schutzschicht gelegene Backingschicht (Stützschicht) umfasst. Diese Backingschicht kann in Form einer Folie oder eines Films ausgestaltet sein. Bevorzugt liegt die Backingschicht als Film vor. Weiterhin ist bevorzugt, dass die Backingschicht Polyurethan (PU) enthält oder daraus besteht. PU bietet den Vorteil, dass es (in Abhängigkeit von der Verarbeitung) geräuscharm oder sogar geräuschlos ist, wohingegen andere synthetische Materialien häufig einen steifen und knisternden Eindruck erzeugen und hierdurch von manchen Patienten als unangenehm wahrgenommen werden.

Alternativ kann die Backingschicht aus einem Polyolefin wie Polyethylen (PE) bestehen oder aus Polyvinylchlorid (PVC) oder Polyester. Durch eine Backingschicht in Form eines Films oder einer Folie kann die Verdunstung aus der Quellschicht erheblich reduziert werden. Dies erlaubt eine längere Verweildauer des Produktes auf der Wunde, ohne dass dessen Funktionalität im Sinne einer feuchten Wundbehandlung und dem für das Produkt charakteristischen Saug-Spül-Mechanismus eine Einschränkung erfahren würde.

Die Backingschicht kann transparent sein. Auf diese Weise kann durch optische Kontrolle ein Eindruck darüber gewonnen werden, ob die Quellschicht bereits die maximal mögliche Menge an Wundexsudat aufgenommen hat und sich damit ein stabiles Fluid-Gleichgewicht zwischen Wunde und dem Wundversorgungsprodukt eingestellt hat. Sollte dies der Fall sein, kann die Entscheidung getroffen werden, einen Verbandswechsel durchzuführen. Das verbrauchte Wundversorgungsprodukt kann dann zusammen mit den aufgenommenen Wundflüssigkeiten, Erregern, gelösten Belägen und anderen daran gebundenen Komponenten entsorgt werden. Nach Anbringen eines neuen, erfindungsgemäßen Wundversorgungsproduktes wird das Spülen der Wunde mit frischer Salzlösung fortgesetzt. Daneben kann eine transparente Backingschicht eine Einschätzung darüber erlauben, ob es zu einer bakteriellen Infektion der Wunde gekommen ist, da eine solche häufig mit einem Farbumschlag des Exsudats einhergeht.

Die Backingschicht soll nach einer bevorzugten Ausführungsform im Wesentlichen die Ausdehnung der Schutzschicht haben. Nach einer weiteren Ausführungsform des Wundversorgungsproduktes überragt die Backingschicht die Schutzschicht und bildet auf diese Weise einen umlaufenden Kleberand. Dies verhindert besonders wirkungsvoll, dass über dem Wundgebiet getragene Kleidungsschichten oder therapeutische Textilien (z.B. Kompressionsstrümpfe oder Bandagen) mit Salzlösung oder Wundexsudat benetzt werden und erlaubt die besonders effiziente Rückabsorption des an die Wunde abgegebenen Anteils der wässrigen Salzlösung zurück an die Quellschicht.

Besonders geeignete Mittel, um das erfindungsgemäße Wundversorgungsprodukt an der Wundstelle zu fixieren, sind Sekundärverbände wie z.B. Klebefolien, Gaze, Bandagen und Rollenpflaster. Unter diesen Mitteln sind Klebefolien bevorzugt, da sie - wie das Wundversorgungsprodukt selbst auch - zugeschnitten werden können, ohne an Funktion einzubüßen oder auszufransen. Weiterhin reduzieren Klebefolien die Verdunstung und behindern auch beim Anbringen an der Fußsohle den Patienten beim Gehen nicht. Darüber hinaus können Klebefolien wasserdicht ausgestaltet werden und dadurch das Wundversorgungsprodukt von unerwünschten Außeneinwirkungen (z.B. beim Duschen) schützen.

Besonders bevorzugt ist das Backing bzw. die Backingschicht elastisch und / oder bedruckbar und / oder undurchlässig für Wasser und Wasserdampf. Als undurchlässig für Wasser und Wasserdampf soll in diesem Sinne ein Material verstanden werden, dessen Moisture Vapor Transmission Rate (MVTR) unterhalb von 1000 g / m2 / 24 h liegt, besser unterhalb von 800 g / m2 / 24 h, noch besser unterhalb von 600 g / m2 / 24 h und am besten unterhalb von 100 g / m2 / 24 h. Gleichzeitig kann vorgesehen sein, dass das Backing wasserdicht gegenüber einer Wassersäule von 800 mm, besser 1.300 mm und am besten 2.000 mm ist. Diese Werte können beispielsweise dadurch erreicht werden, dass das Backing bzw. die Backingschicht PU oder PET enthält oder daraus besteht. Dabei gilt, dass dickere Folien einen niedrigeren MVTR-Wert bieten. Dabei kann die Backingschicht beispielsweise eine Dicke (entspricht im fertigen Produkt in Seitenansicht einer Höhe) von 0,2 mm bis 1 mm oder 0,3 mm bis 0,8 mm aufweisen. Eine eventuelle Klebebeschichtung bleibt hierbei in der Messung unberücksichtigt. Eine MVTR unterhalb von 1000 g / m2 / 24 h kann in der Regel bereits ab einer Schichtdicke von 0,2 mm realisiert werden. Die angegeben MVTR-Werte beziehen sich auf eine wässrige Salzlösung, bevorzugt auf eine isotonische Salzlösung, besonders bevorzugt eine isotonische Ringerlösung.

Der Vliesstoff der proximal gelegenen flüssigkeitsdurchlässigen Schicht und / oder der Schutzschicht kann thermisch verfestigte Fasern enthalten. Auf diese Weise wird die Bindungsfestigkeit erhöht und gleichzeitig können leichtere Vliesstoffe generiert werden, was zu einer Materialeinsparung führt. Gleichzeitig bleibt die Wasserdurchlässigkeit erhalten. Der thermisch verfestigte Vliesstoff kann thermoplastische Fasern enthalten. Beispiele hierfür sind Polypropylen (PP), Polyvinylchlorid (PVC), Polyethylen (PE), Polyester (PET), Polyacryl (PC), Polyacrylnitril (PAN), Polyamid (PA), Polyurethan (PU), Viskose (CV) und Mischungen aus zwei oder mehr dieser Fasertypen. Ferner können thermoplastische Zusatzkomponenten enthalten sein. Ein Beispiel für eine thermoplastische Zusatzkomponente ist PET.

Zur Herstellung eines geeigneten thermisch verfestigten Vliesstoffes können die zu einem lockeren

Vlies gelegten Fasern bis zum Schmelzpunkt erwärmt werden, wodurch sie sich verbinden. Zum Einsatz kommen kann die Kalanderverfestigung (auch Thermobonding genannt) oder die Heißluftverfestigung (auch Thermofusion genannt), wobei im Falle von PP-Fasern sich besonders die Kalanderverfestigung bewährt hat.

Bei Einsatz von Fasern, die sich nicht in Reinform für die thermische Verfestigung eignen, können Bindefasern beigemengt werden, um auf diese Weise dennoch eine thermische Verfestigung zu ermöglichen. So kann das thermisch verfestigte Vlies neben thermoplastischen Fasern und thermoplastischen Zusatzkomponenten auch andere Faserarten wie z.B. Baumwolle oder Viskose enthalten. Bevorzugt handelt es sich bei diesen Fasern um Fasern natürlichen Ursprungs oder teilsynthetische Fasern wie Viskose.

Die Wärmeübertragung für die thermische Verfestigung kann mittels Wärmeleitung, Konvektion oder Strahlung erfolgen. Besonders geeignet sind das Kalander- und das Heißluftverfahren. Diese Art der Verfestigung ermöglicht die Umwandlung eines losen Faservlieses in einen festen, beständigen Vliesstoff. Das Risiko, das Fasern sich lösen und in die Wunde gelangen ist auf diese Weise stark reduziert.

Die SAF in der Quellschicht können ein Polymer umfassen oder aus einem Polymer bestehen, das Acrylat enthält. Es kann sich hierbei um ein Polyacrylamid oder ein Derivat eines Polyacrylamids oder um ein Acrylamid-Copolymer handeln. Gemäß einer bevorzugten Ausführungsform der Erfindung enthalten die SAF in der Quellschicht des Wundversorgungsprodukts ein Polymer, welches quervernetzbar ist. Ein Beispiel für quervernetzbare Polymere, aus denen SAF gewonnen werden können oder die einen Bestandteil von SAF bilden können, sind acrylathaltige Polymere bzw. Acrylat-Co-Polymere.

Die SAF in der Quellschicht des Wundversorgungsprodukts können vorzugsweise ein quervernetztes Polymer enthalten. Bevorzugt beruht die Quervernetzung auf der Bildung von Ester-Verbindungen. Das quervernetzte Polymer kann durch diese Ester-Bindungen quervernetzt oder quervernetzbar sein. Durch den Grad der Quervernetzung kann die Biegsamkeit bzw. der Widerstand der SAF (Biegewiderstandsmoment) in Anwesenheit der wässrigen Salzlösung eingestellt werden. Je höher der Quervernetzungsgrad, desto zäher wird die gelartige Konsistenz der SAF, die in Anwesenheit von Wasser oder anderen wässrigen Flüssigkeiten wie beispielsweise Wundexsudat als Hydrogele vorliegen können. Gleichzeitig steigt mit dem Quervernetzungsgrad die Belastbarkeit der Quellschicht und damit des Wundversorgungsproduktes insgesamt.

Die Quervernetzungsbindungen können Acrylesterbindungen sein. Die Vernetzung kann über radikalische oder kationische Polymerisationsmechanismen oder andere Ver- oder Umesterungsmechanismen wie beispielsweise die Michael-Addition, erfolgen. Bevorzugt wird die radikalische Polymerisation. Polyacrylamide können quervernetzt werden, indem sie in ein saures Milieu eingebracht werden. Je nachdem mit welcher Geschwindigkeit die Reaktion erfolgen soll, kann der pH-Wert hierzu kleiner als 6,0, kleiner als 5,0 oder kleiner als 4,0 sein. Beispielsweise kann der pH-Wert im Bereich von 3,0 bis 6,0 oder 1,0 bis 5,0 oder 0,0 bis 4,0 liegen.

In diesem Zusammenhang können die SAF ein Polymer enthalten, das zu 5 bis 50 % quervernetzt ist, bevorzugt 10 bis 40 %. Bei acrylhaltigen Polymeren kann der Quervernetzungsgrad anhand der Menge der über Ester-Bindungen quervernetzten Acrylgruppen innerhalb des Polymers bestimmt werden. Bei einem Quervernetzungsgrad von 50 % wäre somit die Hälfte der Acrylgruppen quervernetzt.

Ferner können die SAF ein Co-Polymer enthalten, bei dem es sich um ein Acryl-Co-Polymer handeln kann.

Ein mögliches Beispiel ist Poly(Acrylsäure-Co-Acrylamide)/Polyvinylalkohol. Dieser kann direkt zu SAF gesponnen werden.

Die Quervernetzung der SAF steht deren Vernadelung mit mindestens einer anderen Faserart (einer Zusatzfaser, die keine SAF ist) zu einem Nadelfilz nicht entgegen. Die Quervernetzung kann sowohl vor der Vernadelung als auch im Anschluss stattfinden, wobei empfohlen wird, diese vor der Vernadelung vorzunehmen, da auf diese Weise der Quervernetzungsgrad genauer eingestellt werden kann. Eine thermische Verfestigung kann sich an die Vernadelung anschließen, wobei diese drei Maßnahmen (Quervernetzung, Vernadelung und thermische Verfestigung) sich gegenseitig synergistisch ergänzen und in einer Quellschicht resultieren, die hervorragende Feuchtigkeitsspeicherung, Feuchtigkeitsabgabe und ausgezeichnete mechanische Festigkeit selbst nach einem Zuschneiden durch den Endverbraucher oder Pflegekräfte aufweist.

Das Verhältnis zwischen SAF und Zusatzfaser in der Quellschicht kann beispielsweise folgendermaßen aussehen: 20 Gew.-% bis 80 Gew.-% SAF und 80 Gew.-% bis 20 Gew.-% Zusatzfasern. Bevorzugt beträgt der Anteil der SAF in der Quellschicht 20 Gew.-% bis 60 Gew.-%, besonders bevorzugt 20 Gew.-% bis 40 Gew.-%, wobei der übrige Anteil durch die Zusatzfasern gestellt wird. Eine mögliche Ausführungsform enthält 25 Gew.-% SAF und 75 Gew-% Polyolefin-basierte Fasern in der Quellschicht.

Erfindungsgemäß sind die flüssigkeitsdurchlässige Schicht, die Quellschicht und die Schutzschicht vollflächig (also über die gesamte Fläche der jeweiligen Ober- bzw. Unterseite) miteinander verbunden. Die Flächen können adhäsiv miteinander verbunden sein, wobei die adhäsive Verbindung der Flächen miteinander direkt (direkt angrenzend) oder indirekt - z.B. durch das Einfügen weiterer Zwischenschichten wie beispielsweise eine perforierte beidseitige klebende Folie oder eine poröse Schmelzfolie (thermoplastisch) - ausgestaltet sein kann. Die Schichten können auf adhäsive Weise beispielsweise durch einen Kleber bzw. eine Klebeverbindung zusammengefügt bzw. verbunden sein. Die Klebeverbindung bzw. Klebeschicht kann im fertigen Produkt als kohäsive Schicht vorliegen, das heißt innerhalb dieser ziehen die Teilchen des Klebers (Atome, Moleküle etc.) sich gegenseitig an. Bevorzugt handelt es sich um einen Schmelzkleber bzw. eine Schmelzkleberverbindung. Beispiele für mögliche Schmelzkleber sind Co-Polymere und Polyolefin-basierte Schmelzkleber wie Polyester bzw. Polyesterderivate wie Co-Polyester. Derartige Schmelzkleber sind hervorragend geeignet, um Vliesschichten, insbesondere solche welche synthetische Fasern enthalten, dauerhaft zusammenzufügen. Sie sind darüber hinaus mit einer späteren Hitze-Sterilisation in aller Regel technisch kompatibel. Der Schmelzpunkt kann dabei auch unter der späteren Sterilisationstemperatur liegen, da das Produkt während der Sterilisation sich bereits in einer Umverpackung befindet, die dem Fabrikat zusätzliche Stabilität verleiht. So kann der Schmelzpunkt des Schmelzklebers bzw. der Schmelzkleberverbindung beispielsweise bei 45 °C bis 120 °C, bevorzugt bei 45 °C bis 70 °C liegen. Bei Bedarf können - z.B. durch den Einsatz von Co-Polyester - Höchstwerte beim Schmelzpunkt von bis 140 °C und teilweise sogar darüber hinaus erreicht werden. Es wird empfohlen das Wundversorgungsprodukt im feuchten Zustand (also mit der enthaltenen wässrigen Salzlösung) zu sterilisieren, da auf diese Weise niedrige Sterilisationstemperaturen (z.B. 120 °C) bereits ausreichend sein können, sofern für eine ausreichende Einwirkungsdauer gesorgt wird.

Die oben genannten Schmelzkleber können dem Schmelzprozess in unterschiedlichen Ausgangsmaterialien zugeführt werden. So ist es möglich den Schmelzkleber in Form von Partikeln, als Netz oder als Folie bereitzustellen. Die Bereitstellung in Form von Partikeln oder als Netz wird bevorzugt, weil hierdurch die Beweglichkeit des resultierenden Produktes nicht eingeschränkt wird. Partikel bieten zudem den Vorteil der Rieselfähigkeit. Folien und Netze sind besonders geeignet, wenn unterschiedliche Schmelzkleber miteinander kombiniert werden sollen. Dabei können Schmelzkleberkombinationen zu besonders belastbaren Klebeverbindungen führen, wobei einer der verwendeten Kleber beim Herstellungsprozess die jeweiligen Schichten fixiert und der andere Kleber (mit höherem Schmelzpunkt) während des Sterilisationsprozesses reagiert und die Initialverbindung weiter verstärkt, ohne dass zusätzliche thermische Energie während des Prozesses aufgewandt werden müsste.

Die Schichten des Wundversorgungsproduktes - insbesondere die flüssigkeitsdurchlässige Schicht, die Quellschicht und die Schutzschicht - können als Laminat vorliegen, wobei weitere Schichten wie ein Adhäsivträger (proximal) oder ein Backing (distal) ebenfalls eingebunden werden können und dann Teil des Laminats sind. Dazu können diese entweder durch Einsatz von thermischer Energie miteinander verbunden werden und / oder mithilfe von Klebstoffen. Bei Einsatz von Klebstoffen, die nicht erhitzt werden, entsteht ebenfalls ein Laminat, welches in diesem Fall ein Kaltlaminat darstellt. Empfohlen wird zwischen den Schichten den bereits erwähnten Schmelzkleber aufzutragen, um das erhaltene Laminat beständiger zu machen. Die benötigten Temperaturen hängen von den zu behandelnden Materialien ab und können beispielsweise im Bereich von 45 - 160 °C liegen. Normalerweise genügt eine Einwirkungsdauer von 2 Sekunden bis 10 Minuten. Die optimale Einwirkungsdauer variiert in Abhängigkeit von der gewählten Temperatur, wird jedoch in den meisten Fällen im Bereich von 2 bis 30 Sekunden liegen.

Die auf diese Weise adhäsiv zusammengefügten Schichten des Wundversorgungsproduktes haben den Vorteil, dass es zur Ausbildung einer flächigen Verbindung kommt, die auch nach einem möglichen Zuschneiden des Produktes die Schichten weiterhin zusammenhält. Bevorzugt handelt es sich bei der flächigen Verbindung um eine vollflächige adhäsive Verbindung, besonders bevorzugt um eine vollflächige Klebeverbindung und im Optimalfall um eine vollflächige Schmelzkleberverbindung. Umgekehrt führen Methoden, welche die einzelnen Schichten lediglich entlang deren Rändern zusammenfügen (z.B. klassisches Schweißen) nicht zu einem zuschneidbaren Produkt.

Andere Möglichkeiten die Schichten miteinander zu verbinden, bestehen darin, Fasern der einen Schicht in einer angrenzenden Schicht zu verankern. Auf diese Weise können zwei Schichten beispielsweise durch Verweben oder Verfilzen miteinander verbunden sein.

Das Wundversorgungsprodukt lässt sich in zwei oder mehr Segmente zuschneiden. Alle auf diese Weise erhaltenen Segmente sind in der Wundversorgung einsetzbar. Die durch das Zuschneiden erhaltenen Segmente sind in der Lage, die in der Quellschicht eingelagerte wässrige Salzlösung an die Wunde abzugeben und diese dabei zu spülen und zu reinigen.

Ein weiterer Aspekt der Erfindung betrifft ein zuschneidbares flächiges Wundversorgungsprodukt, bei dem die flüssigkeitsdurchlässige Schicht, die Quellschicht und die Schutzschicht flächig über eine Schmelzverbindung zusammengefügt sind und wobei die Schmelzverbindung ein eingeschmolzenes Polyolefin oder eingeschmolzenes Copolymer enthält oder daraus besteht. Ein derartiges Laminat lässt sich vorteilhaft zuschneiden, ohne an den Schnittkanten auszufransen.

Ein besonderer Vorteil des erfindungsgemäßen zuschneidbaren flächigen Wundversorgungsproduktes ist, dass der vom Wundversorgungsprodukt ausgehende Spüleffekt bei wiederholter reversibler (nicht plastischer) Verformung oder bei wiederholter Druckkompression des Wundversorgungsproduktes verstärkt bzw. intensiviert wird. Dies kann in der Praxis erreicht werden durch z.B. Anbringung des Wundversorgungsproduktes an der Fußsohle und anschließender wiederholter Druckbelastung und Druckentlastung beim Gehen durch den Patienten bzw. Endanwender. Auf diese Weise wird beim Auftreten das Wundversorgungsprodukt durch das Körpergewicht komprimiert und der nicht fest an das SAF gebundene Anteil der wässrigen Salzlösung aus dem Wundersorgungsprodukt teilweise herausgedrückt. Sobald der Patient den Fuß hebt und das Wundversorgungsprodukt sich wieder ausdehnt, wird ein Anteil der abgegebenen Salzlösung (zusammen mit gelösten Stoffen, Erregern etc.) wieder aufgenommen. Dieser Vorgang findet während des Gehens in repetitiver Weise statt, was eine besonders intensive Spülung der Wunde ermöglicht.

Eine andere Möglichkeit, beispielsweise bei der Behandlung eines offenen Beines, ist das Anbringen des Wundversorgungsproduktes und anschließendes Umwickeln mit einer Kompressionsbinde. Die Kompressionsbinde dient dabei als Widerlager für die sogenannte Venen-Muskel-Pumpe. Das Wundversorgungsprodukt wird während der Muskelkontraktion zwischen Körperoberfläche und Kompressionsbinde zusammengedrückt. Bei der nachfolgenden Muskelentspannung kann sich das Wundversorgungsprodukt wieder ausdehnen. Die Muskelanspannung entsteht unwillkürlich beim Gehen. Falls ein Patient bettlägerig ist, können alternativ auch im liegenden Zustand geeignete Übungen durchgeführt werden - beispielsweise unter Anleitung eines Physiotherapeuten. Auch in den hier genannten Fällen tritt ein vorteilhafter verstärkter Spüleffekt auf.

Im Sinne dieses Aspekts (verstärkter Spüleffekt) kann der Ausdruck wiederholte reversible Verformung oder wiederholte Druckkompression bedeuten, dass das Wundversorgungsprodukt in der Lage ist, die Wunde verstärkt zu spülen, wenn es mindestens drei Mal verformt oder komprimiert wird und dabei jedes Mal ohne zusätzliche Unterstützung im Wesentlichen in seine Ausgangsform zurückkehrt, wobei eine verstärkte Spülung als eine solche zu verstehen ist, bei der mehr Volumen an wässriger Salzlösung abgegeben wird (z.B. an eine Wunde oder eine Messvorrichtung) im Vergleich zu einem rein passiven Stoffaustausch (ohne Verformung oder Kompression) entlang des Konzentrationsgradienten. Das Wundversorgungsprodukt kann hierbei im Ausgangszustand z.B. zu 80 % der maximalen Absorptionskapazität mit der wässrigen Salzlösung gesättigt sein.

Im Kontext der Erfindung ist das Wundversorgungsprodukt in der Lage während der Wundbehandlung einen muskulär angetriebenen Pumpeffekt zur Spülung der zu behandelnden Wunde mit der in der Quellschicht enthaltenen wässrigen Salzlösung zu erzeugen, und zwar insbesondere beim Gehen, wenn das Produkt an der Fußsohle fixiert ist.

Der auf diese Weise intensivierte Spüleffekt ermöglicht eine äußerst schonende Reinigung der Wunde von Nekrosen und Fibrinbelägen, wodurch in der Regel eine für den Patienten belastende chirurgische invasive Reinigung mittels Skalpell, scharfem Löffel etc. vermieden werden kann.

Bevorzugt stellt der verstärkte Spüleffekt gleichzeitig einen verstärkten Saug-/Spüleffekt dar, der gleichzeitig mit einer verstärkten Aufnahme von aus der Wunde aufgesaugten Stoffen einhergeht. Z.B. werden aus der Wunde ausgespülte Mikroorganismen in der Quellschicht zurückgehalten und beim nächsten Verbandswechsel entfernt.

Weiterhin kann vorgesehen sein, dass nach dem Zuschneiden des erfindungsgemäßen Wundversorgungsproduktes die strukturelle Integrität des verbliebenen, für die Wundversorgung vorgesehenen Bereiches erhalten bleibt, so dass durch die Filzstruktur der Quellschicht keine faser- oder partikelförmigen Bestandteile des zugeschnittenen Wundversorgungsprodukts, insbesondere keine superabsorbierenden Fasern, freigesetzt werden. Ein solches Wundversorgungsprodukt ist somit fusselfrei, und zwar auch nach einem Zuschneiden unmittelbar vor der Anwendung.

Bei dem erfindungsgemäßen Wundversorgungsprodukt kann vorgesehen sein, dass der pH-Wert der wässrigen Salzlösung von weniger als 7,0 durch eine in den superabsorbierenden Fasern enthaltene Säuregruppe, bevorzugt durch Acrylsäure, vermittelt wird. Diese Säuregruppe kann Teil des Polymers sein, welches in den SAF enthalten ist oder aus welchem die SAF bestehen. Bei dem Polymer kann es sich um Polyacrylsäure handeln. Der Vorteil ist, dass auf weitere Bestandteile zur Ansäuerung verzichtet werden kann und ein für die allermeisten Wunden heilungsförderlicher pH-Wert im leicht sauren Bereich eingestellt werden kann. Bevorzugt liegt der durch die besagte Säuregruppe vermittelte pH-Wert der wässrigen Salzlösung im Bereich von 4,5 bis 6,9. Besonders bevorzugt liegt der pH-Wert im Bereich von 5,0 bis 6,5.

Ferner ist bevorzugt vorgesehen, dass die flüssigkeitsdurchlässige Schicht, die Quellschicht und die Schutzschicht bei Flüssigkeitsaufnahme eine im Wesentlichen identische laterale Größenzunahme aufweisen. Im Wesentlichen identische laterale Größenzunahme meint, dass sich die flächige Ausdehnung dieser Schichten in demselben Maße vergrößern, wenn sie Feuchtigkeit aufnehmen, wobei geringe Flächenunterschiede (nach Ausdehnung) von bis zu 5 %, bevorzugt von bis zu 3 % zwischen den jeweils aneinandergrenzenden Schichten möglich und miterfasst sind. Die Ausdehnung kann gemäß dem Standard AATCC TM135 bestimmt werden. Bezüglich einer möglichen Schrumpfung nach Flüssigkeitsabgabe gilt dies analog in Bezug auf die Verkleinerung der Flächen der Schichten.

Der Vorteil einer im Wesentlichen gleichen Flächenveränderung der drei Schichten bei Kontakt mit Flüssigkeit ist, dass es in Folge der Feuchtigkeitsaufnahme nicht zu einer Verformung (z.B. Krümmung oder Ausstülpung) des Wundversorgungsproduktes kommt und die planare Form erhalten bleibt.

In diesem Kontext können die flüssigkeitsdurchlässige Schicht, die Quellschicht und die Schutzschicht auch denselben oder im Wesentlichen denselben thermischen lateralen Ausdehnungskoeffizienten haben, wobei sich dieser auf die Flächenausdehnung bezieht. Unterschiede von bis zu 5 %, bevorzugt von bis zu 3 % zwischen den jeweils aneinandergrenzenden Schichten sind als Toleranzen miterfasst. Ein identischer oder im Wesentlichen identischer thermischer lateraler Ausdehnungskoeffizient bietet einen Vorteil bei einer Hitze-Sterilisation des Wundversorgungsproduktes. Andernfalls kann es zu unerwünschten Verformungen des Produktes während der Sterilisation kommen und die Sterilisation muss mit anderen (in der Regel aufwändigeren oder teureren) Verfahren durchgeführt werden, beispielsweise Bestrahlung oder Begasung mit Ethylenoxid.

Eine identische oder im Wesentlichen identische Ausdehnung bzw. thermische Ausdehnung kann dadurch erreicht werden, dass mehreren Schichten Materialien zum Einsatz kommen, die dasselbe oder ein sehr ähnliches Ausdehnungsverhalten zeigen. So zeigen synthetische Fasern sowohl untereinander als auch im Vergleich zu Baumwolle ein sehr ähnliches Ausdehnungs- und Schrumpfungsverhalten.

Erfindungsgemäß ist vorgesehen, dass die Quellschicht des Wundversorgungsproduktes neben SAF mindestens eine andere Faserart bzw. mindestens eine Zusatzfaser umfasst. Hierbei handelt es sich vorteilhaft um eine zellulosehaltige Faser wie zum Beispiel Lyocell, um eine Polyolefin-basierte Faser, eine polyesterhaltige Faser, bevorzugt Polyethylenterephthalat und / oder eine polyamidhaltige Faser.

Lyocell-Fasern haben den Vorteil eines hervorragenden Feuchtigkeitsspeicherungsvermögens, welches die bereits hohe Kapazität von Baumwolle noch übertrifft. Somit unterstützen Lyocell-Fasern die SAF in synergistischer Weise, verleihen der Quellschicht die nötige strukturelle Belastbarkeit und halten die SAF innerhalb der Quellschicht an Ort und Stelle.

Weiterhin ist es im Hinblick auf das erfindungsgemäße Wundversorgungsprodukt möglich, dass der Vliesstoff der flüssigkeitsdurchlässigen Schicht und / oder der Schutzschicht Viskose und Polyester enthält oder aus diesen Polymeren besteht. Dabei kann der Polyesteranteil im Vliesstoff beispielsweise 30 bis 50 Massenprozent betragen. Durch die Beimischung des hydrophoben Polyesters entsteht sowohl ausgehend von der flüssigkeitsdurchlässigen Schicht als auch der Schutzschicht ein hygroskopischer Gradient, der Flüssigkeit durch die beiden zuletzt genannten Schichten in Richtung der Quellschicht durchleitet. Gleichzeitig kann die wässrige Salzlösung von der Quellschicht kommend die flüssigkeitsdurchlässige Schicht passieren und dann in die Wunde gelangen, ohne in der flüssigkeitsdurchlässigen Schicht zurückgehalten zu werden.

Ferner ist es möglich, dass die flüssigkeitsdurchlässige Schicht und die Schutzschicht aus einem identischen Material oder einer identischen Materialmischung bestehen und / oder dasselbe Flächengewicht haben. Dabei kann es sich um solche Materialien oder Materialgemische handeln, die im letzten Absatz aufgeführt sind. Die Verwendung identischer Materialien für beide Schichten kann den Produktionsprozess vereinfachen und beschleunigen, da beide Schichten vom selben Maschinentypen prozessiert werden können.

Generell ist möglich, dass der Vliesstoff der flüssigkeitsdurchlässigen Schicht und / oder der Schutzschicht im trockenen Zustand ein Flächengewicht von 15 bis 50 g/m2, beispielsweise 18 bis 45 g/m2, hat. Das Flächengewicht kann anhand der Norm DIN EN 12127 bestimmt werden. Es hat sich gezeigt, dass derart beschaffene Schichten eine sehr gute strukturelle Belastbarkeit mit einer hohen Durchlässigkeit für Flüssigkeiten kombinieren. Die Schichten bleiben auch nach einem möglichen Zuschneiden belastbar, behindern jedoch nicht den gewünschten Saug-/Spüleffekt des Wundversorgungsproduktes.

Je nach vorgesehenem Anwendungsgebiet kann es vorteilhaft sein, den Vliesstoff der flüssigkeitsdurchlässigen Schicht proximal mit einem Silikon oder silikonhaltigen Material auszustatten oder zu beschichten. In diesem Sinne kann die flüssigkeitsdurchlässige Schicht des erfindungsgemäßen Wundversorgungsproduktes an ihrer proximalen Seite zumindest partiell mit Silikon oder silikonhaltigem Material beschichtet sein. Diese Substanzen können auf den Vliesstoff der flüssigkeitsdurchlässigen Schicht (proximale Seite) aufgetragen werden. Bei dem silikonhaltigen Material kann es sich beispielsweise um einen silikonbasierten Klebstoff handeln oder um ein Silikongel, welches nach dem Auflegen nicht aushärtet und hierdurch adhäsive Eigenschaften erhält.

In diesem Sinne umfasst die Erfindung eine Version des Wundversorgungsproduktes, wobei das Wundversorgungsprodukt über eine zusätzliche silikonhaltige Wundkontaktschicht verfügt, welche direkt oder in Form einer beschichteten Folie an die Außenseite der flüssigkeitsdurchlässigen Schicht (also in proximaler Richtung) angebracht ist.

Es wird nicht empfohlen das fertige Wundversorgungsprodukt mit einer vollflächig bedeckten bzw. beschichteten flüssigkeitsdurchlässigen Schicht auszustatten, da dies aufgrund der hydrophoben Eigenschaften des Silikons den Flüssigkeitsaustausch behindern würde. Stattdessen kann der Auftrag musterförmig erfolgen - z.B. in Streifen bzw. streifenförmig. Alternativ kann der Auftrag vollflächig erfolgen und das Silikon bzw. silikonhaltige Material kann im Anschluss perforiert werden. Diese vollflächige Beschichtung mit Perforationen bietet gegenüber dem musterförmigen Auftragen den Vorteil, dass unabhängig von der Art eines späteren Zuschneidens die Randbereiche der flüssigkeitsdurchlässigen Schicht immer beschichtet sind und somit der gesamte Rand dieselbe Höhe hat.

Alternativ kann das Silikon oder silikonhaltige Material über eine Folie oder einen Film an der flüssigkeitsdurchlässigen Schicht angebracht werden. Die Folie oder der Film können PU enthalten oder daraus bestehen. Sie sind an der distalen Seite mit einem Klebstoff wie einem Acrylkleber beschichtet. Die mit Klebstoff beschichtete Folie oder der mit Klebstoff beschichtete Film wird in Kontext dieser Erfindung auch als Adhäsivträger bezeichnet. Der distal gelegene Klebstoff muss nicht zwingend hautkompatibel sein. Die gegenüberliegende Seite von Folie oder Film (proximale Seite) sind mit dem erwähnten Silikon oder silikonhaltigen Material beschichtet. Die Anwendung vom Film oder Folie bietet den Vorteil, dass dieser als Massenware (z.B. Rollmaterial) vorrätig gehalten werden kann und während des Herstellungsverfahrens bei Bedarf schnell und automatisiert an die proximale Seite der flüssigkeitsdurchlässigen Schicht angebracht werden kann. Film oder Folie bilden mit der flüssigkeitsdurchlässigen Schicht eine stabile Verbindung aus. Wenn der Film oder die Folie vollflächig auf der proximalen oder distalen Seite beschichtet ist, wird empfohlen den Adhäsivträger vor Applikation auf die flüssigkeitsdurchlässige Schicht mit durchgehenden Perforationen auszustatten. Durchgehend meint, dass die Perforationen sowohl den Film oder die Folie als auch die beiden Beschichtungen durchdringen und ein nachfolgender Stoffaustausch über die flüssigkeitsdurchlässige Schicht durch die Perforationen ermöglicht wird.

Weiterhin kann der derart ausgestaltete Adhäsivträger vollflächig oder musterförmig (z.B. in Form von Streifen) an der flüssigkeitsdurchlässigen Schicht angebracht sein.

In diesem Sinne umfasst die Erfindung ein Wundversorgungsprodukt umfassend eine silikonhaltige Wundkontaktschicht, die die flüssigkeitsdurchlässige Schicht lediglich teilweise überlagert und wobei die silikonhaltige Wundkontaktschicht bevorzugt perforiert ist.

Falls das Silikon bzw. das silikonhaltige Material oder die Folie perforiert werden soll, ist auf eine ausreichende Größe der Perforationen zu achten. So kann eine Perforation eine Fläche von 0,05 mm2 bis 7,00 mm2 haben, wobei größeren Perforationsflächen der Vorzug zu geben ist. So kann vorgesehen sein, dass die Durchschnittsfläche pro Perforation 0,8 mm2 bis 7,00 mm2 oder 0,8 mm2 bis 6,00 mm2 beträgt.

Die Perforationen können unterschiedliche Formen bzw. Umrisse haben, wobei kreisförmige oder ovale Perforationen den Vorteil bieten, dass sie eine gute Belastbarkeit beim Dehnen oder Biegen des Wundversorgungsproduktes zeigen, ohne einzureißen. Gleichzeitig bietet das Fehlen von Kanten oder Ecken ein angenehmeres Hautgefühl und reduziert das Irritationsrisiko für den Wundbereich.

Vorteilhaft ist eine offene (unbeschichtete) Fläche der flüssigkeitsdurchlässigen Schicht im Bereich von 10 % bis 25 % oder 12 % bis 23 % ihrer Gesamtfläche. Die offene Fläche kann durch die bereits erwähnten Perforationen erreicht werden oder aber durch musterförmige Auftragung der Beschichtung.

Empfohlen wird, die Beschichtung möglichst dünn aufzutragen, da hierdurch Material eingespart wird, und zwar ohne dass es zu Qualitätseinbußen kommt. Gute Ergebnisse werden erzielt mit einer Beschichtungsmenge von 100 g/m2 bis 200 g/m2, bevorzugt 120 g/m2 bis 175 g/m2 und besonders bevorzugt 135 g/m2 bis 160 g/m2. Die Angaben gelten für die Beschichtung von sowohl Vliesmaterial als auch Folie und zwar vor einer möglichen Perforation. Bei einem musterförmigen Auftrag können die angegebenen Bereiche für die Beschichtungsmenge entsprechend geringer ausfallen und um z.B. 25% reduziert sein.

Bei dem Silikon oder dem silikonhaltigen Material, mit welchem der Vliesstoff oder die Folie beschichtet werden kann, handelt es sich beispielsweise um einen Silikonkleber bzw. um einen Silikon-haltigen Klebstoff oder ein Silikongel. Derartige Klebstoffe bzw. Gele bieten den Vorteil, dass sie das Wundversorgungsprodukt auf der Wunde oder dem Wundbereich fixieren können und dabei atraumatisch sind. Dies heißt, sie lassen sich schmerzfrei, und ohne neu entstandenes Gewebe zu beschädigen, entfernen.

Das erfindungsgemäße Wundversorgungsprodukt kann unterschiedliche äußere Formen und Dimensionen haben. Für die Behandlung einer (äußeren) Wunde wird eine Form empfohlen, die beidseitig (also sowohl proximal als auch distal) flach ist. Für die Behandlung von Ulcera an der Fußsohle ist darüber hinaus vorteilhaft, das Wundversorgungsprodukt mit einer geringen Höhe auszugestalten. Eine solche Wundversorgung stört den Patienten kaum oder gar nicht in seinem üblichen Tagesablauf, da der Patient das Produkt unter Socken und in Schuhen tragen und sogar damit gehen kann. Eine solche geringe Höhe beträgt 1,5 mm bis 6 mm. Innerhalb dieser Spanne liegt auch eine Ausführungsform des Wundversorgungsproduktes, welche über einen Adhäsivträger (proximal) und ein Backing (distal) verfügt. Sollte das Wundversorgungsprodukt lediglich den Mindestaufbau aus Schutzschicht, Quellschicht, flüssigkeitsdurchlässiger Schicht und optionalen Klebeschichten aufweisen, kann die Höhe noch vorteilhaftere 1,7 mm bis 3 mm, bevorzugt 1,7 mm bis 2,5 mm und besonders bevorzugt 1,8 mm bis 2,2 mm betragen. Die angegebenen Bereichsangaben für die Höhe beziehen sich auf den in der vorliegenden Anmeldung beschrieben trockenen Verbund (also ohne wässriger Salzlösung) aus flüssigkeitsdurchlässiger Schicht, Quellschicht und Schutzschicht, und gleichfalls ohne optionale Release-Liner oder sonstiges Verpackungsmaterial. Die Höhe des mit der wässrigen Salzlösung behandelten Produktes kann (in Abhängigkeit von dem beaufschlagten Volumen) im Vergleich zu dem trockenen Produkt üblicherweise um etwa 10 % zunehmen. Folglich hätte das Wundversorgungsprodukt im feuchten Zustand beispielsweise eine Höhe von 1,7 mm bis 6,6 mm, besser 1,9 mm bis 3,3 mm, noch besser 1,9 mm bis 2,8 mm und am besten 2 mm bis 2,4 mm.

Beispiele für weitere mögliche Formen sind kissenförmig und zylinderförmig. Die kissenförmige Variante sorgt für einen besonders guten Polsterungseffekt. Dies wird beispielsweise im Falle von am Knöchel befindlichen Wunden von Patienten als besonders angenehm empfunden. Die zylinderförmige Variante ist besonders vorteilhaft bei tunnelförmigen Wunden und Cavitäten, wobei ein eventuell abstehendes (aus der Wunde herausragendes) Ende einfach abgeschnitten werden kann. Auf diese Wiese wird ein vorschneller oberflächlicher Wundverschluss mit darunterliegender Einkapselung und nachfolgender Abszessbildung verhindert.

Was die Form angeht, so kann das Wundversorgungsprodukt in der Aufsicht rund, rechteckig, quadratisch, oval oder rautenförmig sein. Die Fläche kann beispielsweise 50 cm2 bis 500 cm2 betragen, wobei größere Flächen den Vorteil bieten, auf die jeweilige Wundgröße zugeschnitten werden zu können.

Die Quellschicht des Wundversorgungsproduktes enthält vorzugsweise die wässrige Salzlösung in einer Menge, die ihre Absorptionskapazität nicht ausschöpft. Das heißt, dass die Quellschicht vorzugsweise nicht gesättigt bzw. ungesättigt ist. Auf diese Weise kann die Quellschicht bzw. das Wundversorgungsprodukt weitere Flüssigkeit wie z.B. Wundexsudat oder Blut aufnehmen. So ist es möglich, dass die Quellschicht oder alternativ das ganze Wundversorgungsprodukt zu maximal 50%, maximal 60%, maximal 70%, maximal 80%, maximal 90% oder maximal 95% mit der wässrigen Salzlösung gesättigt ist.

Welcher von diesen Werten am besten geeignet ist, hängt von der zu versorgenden Wundart ab. Je stärker eine Wunde mit Belegen (z.B. Fibrinbelegen), nekrotischem Gewebe oder Biofilm bedeckt ist, desto mehr wird diese Wunde von einer intensiven Spülung profitieren, so dass sich in einem solchen Fall hohe Sättigungswerte von 80% oder mehr empfehlen. Hingegen können stark exsudierende, nicht infizierte Wunden mit solchen Varianten des Produktes behandelt werden, die eine geringere Sättigung im Bereich von 50% bis 70% aufweisen.

Die angegeben Sättigungswerte sollen nachfolgend an einem Beispiel erklärt werden: Wenn die Quellschicht maximal 100 ml einer wässrigen Salzlösung (osmotischer Wert im isotonischen Bereich, also 9 g NaCl auf einen Liter H2O) absorbieren kann, so ist die Quellschicht nach Aufnahme von 50 ml zu 50% gesättigt. Weiterhin können die angegeben Sättigungswerte sich auch auf ein Wundversorgungsprodukt mit den drei Hauptschichten (Schutzschicht, Quellschicht, flüssigkeitsdurchlässige Schicht und dazwischenliegende Klebeschichten) beziehen oder auf das gesamte Wundversorgungsprodukt, welches je nach Bedarf auch über zusätzliche Schichten (z.B. Backing-Schicht) verfügen kann.

Ferner kann bestimmt sein, dass das Wundversorgungsprodukt nicht geeignet oder kompatibel zur Unterdrucktherapie ist, da es beispielsweise keinen Port hat, über welchen ein Unterdruck an das Produkt bzw. eine Wunde unterhalb des Produktes angelegt werden kann.

Im Rahmen der vorliegenden Erfindung kann vorgesehen sein, dass das Wundversorgungsprodukt keine superabsorbierenden Partikel enthält. Bevorzugt sind überhaupt keine Partikel enthalten, die nach dem Zuschneiden des Produktes herausfallen könnten. Ausgenommen hiervon sind Klebestoffpartikel, die während der Herstellung des Produktes eingesetzt werden können, da sich diese mit den übrigen Materialien verbinden und dem gewünschten Effekt der Zuschneidbarkeit nicht entgegenstehen. Auch Wirkstoffpartikel, die sich in der wässrigen Salzlösung auflösen, sind nicht vom Erfindungskonzept ausgenommen.

Bei dem in der wässrigen Salzlösung vorhanden Salz kann es sich um NaCl handeln. Bevorzugt ist die wässrige Salzlösung isotonisch. Eine solche isotonische Salzlösung hat im Wesentlichen denselben osmotischen Druck wie menschliches Blut. Geringe Abweichungen des Druckwerts von 1% in Pascal nach oben bzw. unten sind dabei tolerierbar und fallen ebenfalls unter den Begriff der isotonischen Lösung.

Die wässrige Salzlösung kann über NaCl hinaus auch andere Salze wie KCL oder Salzkombinationen wie beispielsweise KCI und NaCl enthalten. Eine bevorzugte Variante der wässrigen Salzlösung ist die sogenannte Ringerlösung, welche neben NaCl und KCI auch CaCl enthält und isotonisch ist.

Eine isotonische Lösung bietet den Vorteil, dass die ungeschützten Zellen im offenen Bereich der zu behandelnden Wunde keinem osmotischen Stress ausgesetzt werden. Vielmehr wirkt sich die Zufuhr der in der Salzlösung enthaltenen Mineralstoffe positiv auf den Zellmetabolismus aus. So sind menschliche Zellen in der Lage über membranständige Proteine wie beispielsweise die Aquaporine Mineralien aus der Umgebung aufzunehmen.

In diesem Sinne kann die wässrige Salzlösung NaCl, KCI und CaCl enthalten.

Die in der Quellschicht eingelagerte wässrige Salzlösung kann von der Quellschicht in die übrigen Schichten des Wundversorgungsproduktes übertreten. Insbesondere kann die wässrige Salzlösung in der Schutzschicht und der flüssigkeitsdurchlässigen Schicht vorhanden sein. Dies beeinträchtigt die Funktionsweise des Produktes nicht. Ein Durchtritt der Lösung durch die flüssigkeitsdurchlässige Schicht spätestens nach Applikation auf die Wunde ist sogar erwünscht. Bei der Bestimmung der zur Benetzung der Quellschicht notwendigen Lösungsmenge kann die Aufnahmekapazität der übrigen Schichten berücksichtigt werden.

Im Folgenden soll die Erfindung anhand von exemplarischen Zeichnungen näher erläutert werden, wobei die dargestellten Varianten je nach Einsatzzweck mithilfe der in diesem Dokument enthaltenen technischen Informationen abgewandelt werden können.
Figur 1 zeigt eine Ausführungsform des erfindungsgemäßen Wundversorgungsproduktes, welche auf eine Wunde und / oder Haut aufgeklebt werden kann und zudem über ein flüssigkeitsdichtes Backing verfügt, im Querschnitt. Diese Variante eignet sich u.a. hervorragend für die Behandlung von Ulcera an der Fußsohle.
Figur 2 zeigt das Produkt von Figur 1 in zugeschnittener Form in der Aufsicht.

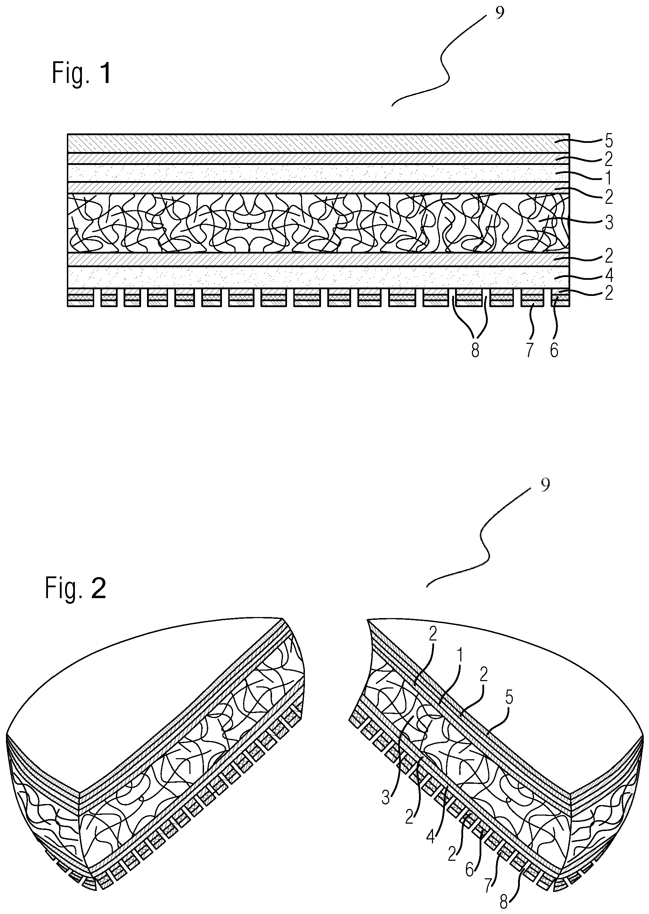

In den Figuren 1 und 2 ist ein erfindungsgemäßes Wundversorgungsprodukt (9) abgebildet, umfassend ein oben gelegenes Backing (5), welches den Austritt der wässrigen Salzlösung (nicht dargestellt) aus der Quellschicht (3) in distaler Richtung minimiert und gleichzeitig als Stützschicht für das Wundversorgungsprodukt (9) fungiert. Das Backing (5) ist mittels einer Klebstoffschicht (2) an der Schutzschicht (1) aus Vliesstoff befestigt. Die Schutzschicht (1) ist mit der Quellschicht (3) mittels einer Klebstoffschicht (2) verbunden. Unterhalb der Quellschicht (3) ist die proximal gelegene flüssigkeitsdurchlässige Schicht (4) mittels einer Klebstoffschicht (2) befestigt. Die flüssigkeitsdurchlässige Schicht (4) wird in proximaler Richtung von einem perforierten Adhäsivträger überlagert. Dieser besteht aus einer Klebstoffschicht (2), mittels welcher die Verbindung zur flüssigkeitsdurchlässigen Schicht (4) gebildet wird, einer Folie aus PU (6) und einer Schicht aus atraumatischem Silikonkleber (7). Der perforierte Adhäsivträger ist über seine gesamte Fläche mit Perforationsöffnungen (8) versehen, welche durch den Silikonkleber (7), den PU-Film (6) und die Klebstoffschicht (2) hindurchreichen. Das Produkt kann mittels des Silikonklebers (7) an der Haut, an der Wunde oder an den Wundrändern fixiert werden, wobei PU-Folie und Silikonkleber als Wundkontaktschicht fungieren. In der Darstellung von Figur 2 ist das Wundversorgungsprodukt (10) mittig in zwei Hälften (Segmente) zerschnitten. Die strukturelle Einheit sowie Funktionalität einer jeden Hälfte bleiben gewahrt.

### Beispiele

Nachfolgend wird die Erfindung anhand von Ausführungsbeispielen näher erläutert:

### Beispiel 1: Herstellung eines adhäsiven Wundversorgungsproduktes zum Anbringen auf die Hautoberfläche

Zunächst wurde eine Quellschicht (3) als Meterware bereitgestellt. An der Unterseite der Quellschicht (3) wurde eine proximal gelegene flüssigkeitsdurchlässige Schicht (4) in Form von Vliesstoff aus PP angebracht. An der Oberseite der Quellschicht (3) wurde eine distal gelegene Schutzschicht (1) angebracht, welche ebenfalls aus einem PP-Vliesstoff bestand. Zur Befestigung der Schichten aneinander wurden Klebepartikel in Form von biologisch abbaubarem Polyester zwischen den Schichten aufgetragen. Die Klebepartikel wurden in einem nachfolgendem thermischen Verfestigungsprozess aneinander befestigt. Dies geschah mittels thermischer Behandlung in einem Trockenofen bei einer Temperatur von mehr als 120°C mit anschließender Walzenkompression. Die verschmolzenen Klebepartikel bildeten jeweils eine Klebeschicht (2). Das derart erzeugte und aus drei Schichten bestehende Laminat hatte ein Flächengewicht von 260 g/m2 bei einer Dicke von 2,3 mm.

Dieses Laminat wurde auf eine Rolle aufgewickelt und diese in eine Convertingmaschine eingehängt. Im nächsten Schritt wurde eine Backingschicht (5) aus PU mittels eines Acrylatklebstoffs auf die obere Seite (bei Anwendung des Wundversorgungsproduktes die distale Seite) des Laminats aufgebracht. Zudem wurde proximal auf die flüssigkeitsdurchlässige Schicht ein perforierter Adhäsivträger aufgebracht. Dieser Adhäsivträger bestand aus einer PU-Folie (6), dessen Unterseite mit hautkompatiblem und atraumatischem Silikonkleber (7) beschichtet war. Die Oberseite der PU-Folie war mit einem Acrylatklebstoff (2) beschichtet, der die PU-Folie mit der flüssigkeitsdurchlässigen Schicht verband. PU-Folie und Acrylatklebstoff bildeten zusammen den Adhäsivträger. Silikonkleber (7) und PU-Folie (6) bildeten die Wundkontaktschicht. Durch die im Adhäsivträger und im Silikonkleber vorhandenen Perforationsöffnungen (8) war sichergestellt, dass ein späterer Durchtritt von Flüssigkeit gewährleistet war.

Um das Silikon (7) zu schützen, wurde dieses mit einem Release-Liner überlagert, der unmittelbar vor dem Einsatz des Produktes an einer Wunde zu entfernen ist. Die Backingschicht (5) war mit einem Aufdruck versehen, der die Aufgabe hatte, dem späteren Anwender zu verdeutlichen, dass es sich um die Oberseite des Produktes handelt, um auf diese Weise die korrekte Anwendung zu erleichtern.

Im nächsten Schritt wurden quadratische Ausschnitte mit einer Fläche von 10 cm x 10 cm aus dem Laminat herausgetrennt. Anschließend wurden die herausgetrennten Produkte mittels einer Verpackungsmaschine prozessiert. In der Verpackungsmaschine wurden die bis dahin trockenen Produkte auf einer flüssigkeitsdichten Verpackungsfolie ausgebreitet. Es wurde wässrige Salzlösung hinzugefügt, und zwar in einer Menge, die die maximale Absorptionskapazität der Produkte nicht ausschöpfte (ca. 31 ml). Es folgte die Versiegelung mit einer oberen Verpackungsfolie (flüssigkeitsdicht), so dass die Wundversorgungsprodukte (9) zwischen unterer und oberer Folie verschlossen waren. Danach wurde der so entstandene Beutel mit den darin enthaltenen Produkten bei 120°C dampfsterilisiert. Anschließend wurden die nun feuchten Wundversorgungsprodukte (9) im Beutel, der gleichzeitig als Teil des Verpackungsmaterials fungierte, steril vorrätig gehalten. Die so erhaltenen sterilen Produkte können nach der Entnahme aus dem Beutel auf eine zu behandelnde Wunde aufgebracht werden.

### Beispiel 2: Zuschneiden der Wundversorgungsprodukte

Das Wundversorgungsprodukt (9) gemäß Beispiel 1 wurde jeweils ca. in der Mitte (d.h. etwa 5 cm entfernt vom linken bzw. rechten Rand) mit einer handelsüblichen Schere in zwei Hälften geschnitten. Der Schnittvorgang konnte mühelos und ohne besonderen Kraftaufwand durchgeführt werden. Die Schnittkanten wurden visuell beurteilt. Es konnten keinerlei Ausfransungen oder lose Fasern festgestellt werden. Die Schnittflächen waren eben, glatt und geradlinig.

Darüber hinaus bildeten die Schichten der Zuschnitte weiterhin einen stabilen Verbund und zeigten keinerlei Tendenz zu Trennung, Zerfall oder Delaminierung. Somit war jeder einzelne Zuschnitt geeignet, um in der Wundversorgung eingesetzt zu werden.

### Beispiel 3: Messung der maximalen Druckbelastung

Es wurde ein etwa zwei Wochen altes feuchtes Wundversorgungsprodukt (9) gemäß Beispiel 1 - jedoch kreisförmig - bereitgestellt und in einen handelsüblichen transparenten Plastik-ZIP-Beutel gelegt. Der Beutel wurde verschlossen und für den weiteren Versuchsablauf in ein Kalibriergerät der Firma Sensomative eingelegt. Das (in der Aufsicht) radiäre Wundversorgungsprodukt (9) hatte einem Durchmesser von 4,5 cm. Anschließend wurden Belastungszyklen gefahren, bei denen jeweils innerhalb von 1 Sekunde eine Gewichtskraft von 188.5 N (entsprechend etwa 19 kg) auf die gesamte Fläche des Produkts (9) aufgebaut wurde. Die Gewichtskraft wurde für die Dauer von einer Sekunde aufrechterhalten und anschließend innerhalb von 2 Sekunden auf 0 N reduziert. Die Anzahl der Belastungszyklen betrug zehn. Der Vorgang fand bei einer Temperatur zwischen 22 °C und 23 °C statt. Die Übertragung der Gewichtskraft fand mithilfe einer im Kalibriergerät verbauten aufblasbaren Hülle aus synthetischem, luftdichten Material statt. Die Hülle wurde während der Belastungszyklen pneumatisch befüllt.

Nach Beendigung des Belastungstests wurde das Produkt (9) aus der Anlage entfernt. Sowohl das Produkt (9) als auch der Beutel wurden visuell inspiziert. Es konnte kein Materialbruch festgestellt werden. Sämtliche Schichten des Produktes (9) waren strukturell intakt. Nach der Entnahme des Produktes (9) aus dem Beutel waren weder am Produkt (9) gerissene Fasern oder ausgetretenes Gel sichtbar noch konnten abgetrennte oder ausgetretene Materialien im Beutelinneren festgestellt werden. Lediglich eine geringe Menge der wässrigen Salzlösung ist im Beutel zurückgeblieben.

## Patentansprüche

1. Zuschneidbares Wundversorgungsprodukt (9), enthaltend
a) eine proximal gelegene flüssigkeitsdurchlässige Schicht (4) mit einem Vliesstoff,
b) eine distal gelegene Schutzschicht (1) mit einem Vliesstoff,
c) eine zwischen der proximal gelegenen flüssigkeitsdurchlässigen Schicht (4) und der distal gelegenen Schutzschicht (1) gelegene Quellschicht (3) in Form eines Vliesstoffes mit superabsorbierenden, polymerhaltigen Fasern, wobei die superabsorbierenden Fasern mit mindestens einer anderen Faserart in einem Filz vorliegen,
d) eine wässrige Salzlösung, die in der Quellschicht (3) eingelagert ist, im eingelagerten Zustand einen pH < 7,0 hat und die während einer Wundbehandlung an eine Wunde abgegeben werden kann,
wobei die proximal gelegene flüssigkeitsdurchlässige Schicht (4), die Quellschicht (3) und die distal gelegene Schutzschicht (1) einen identischen Zuschnitt haben und ohne Überstand vollflächig aneinanderliegen, und wobei das Wundversorgungsprodukt (9) nahtlos ausgestaltet ist.

2. Zuschneidbares Wundversorgungsprodukt (9) nach Anspruch 1, wobei das Wundversorgungsprodukt (9) eine Druckresistenz gegenüber einem Druck von mindestens 8 kg / 100 cm² aufweist, so dass durch Einwirkung eines solches Drucks das Wundversorgungsprodukt (9) lediglich elastisch verformt wird.

3. Zuschneidbares Wundversorgungsprodukt (9) nach Anspruch 1 oder 2, wobei die andere Faserart ausgewählt ist aus der Gruppe bestehend aus: zellulosehaltige Faser, polyesterhaltige Faser, bevorzugt Polyethylenterephthalat und / oder Polyamid und wobei die andere Faserart mit den superabsorbierenden, polymerhaltigen Fasern vernadelt ist, so dass sie in einem Nadelfilz vorliegt.

4. Zuschneidbares Wundversorgungsprodukt (9) nach einem der vorhergehenden Ansprüche, wobei der Vliesstoff der proximal gelegenen flüssigkeitsdurchlässigen Schicht (4) und / oder der distal gelegenen Schutzschicht (1) thermisch verfestigte Fasern enthält.

5. Zuschneidbares Wundversorgungsprodukt (9) nach dem vorhergehenden Anspruch 4, wobei die thermisch verfestigten Fasern ein oder mehrere Polymere ausgewählt aus Polypropylen, Poly-Vinyl-Chlorid, Polyethylen, Polyethylenterephthalat, Polycarbonat, Polyamid, Polyurethan, Polystyrol, sowie Mischungen daraus, enthalten oder daraus bestehen.

6. Zuschneidbares Wundversorgungsprodukt (9) nach einem der vorhergehenden Ansprüche umfassend zusätzlich eine distal zur Schutzschicht (1) gelegene Backingschicht (5), welche vorzugsweise Polyurethan enthält.

7. Zuschneidbares Wundversorgungsprodukt (9) nach Anspruch 6, wobei die Backingschicht (5) elastisch, bedruckbar und flüssigkeitsundurchlässig ist und wobei die MVTR unterhalb von 1000 g/m²/24h liegt.

8. Zuschneidbares Wundversorgungsprodukt (9) nach einem der vorhergehenden Ansprüche, wobei die superabsorbierenden Fasern in der Quellschicht (3) ein Polymer, bevorzugt ein acrylathaltiges Polymer oder ein Acrylat-Co-Polymer, enthalten, welches quervernetzbar ist, wobei die Quervernetzbarkeit bevorzugt auf der Bildung von Ester-Verbindungen beruht.

9. Zuschneidbares Wundvesorgungsprodukt nach einem der vorhergehenden Ansprüche, wobei die proximal gelegene flüssigkeitsdurchlässige Schicht (4), die Quellschicht (3) und die distal gelegene Schutzschicht (1) als Laminat vorliegen.

10. Zuschneidbares Wundversorgungsprodukt (9) nach einem der vorhergehenden Ansprüche, wobei die proximal gelegene flüssigkeitsdurchlässige Schicht (4), die Quellschicht (3) und die distal gelegene Schutzschicht (1) flächig unlösbar miteinander verbunden sind, wobei die Schichten vorzugsweise durch einen Schmelzkleber zusammengefügt sind, und wobei der Schmelzkleber vorzugsweise ein Polyolefin oder Copolymer enthält oder daraus besteht.

11. Zuschneidbares Wundversorgungsprodukt (9) nach einem der vorhergehenden Ansprüche, wobei beim Zuschneiden die strukturelle Integrität des Wundversorgungsproduktes (9) erhalten bleibt, so dass durch die Filzstruktur der Quellschicht keine faser- oder partikelförmigen Bestandteile des Wundversorgungsproduktes (9), insbesondere keine superabsorbierenden Fasern, freigesetzt werden.

12. Zuschneidbares Wundversorgungsprodukt (9) nach einem der vorhergehenden Ansprüche, wobei das Wundversorgungsprodukt (9) über eine zusätzliche silikonhaltige Wundkontaktschicht (7) verfügt, welche direkt oder in Form einer beschichteten Folie (6) an die Außenseite der flüssigkeitsdurchlässigen Schicht (4) angebracht ist.

13. Zuschneidbares Wundversorgungsprodukt (9) nach Anspruch 12, wobei die silikonhaltige Wundkontaktschicht (7) die flüssigkeitsdurchlässige Schicht (4) lediglich teilweise überlagert und bevorzugt perforiert ist.

14. Zuschneidbares Wundversorgungsprodukt (9) nach einem der vorhergehenden Ansprüche, wobei der Vliesstoff der ersten proximal gelegenen flüssigkeitsdurchlässigen Schicht (4) und / oder der Schutzschicht (1) im trockenen Zustand ein Flächengewicht von 15 g/m² bis 50 g/m² hat.

15. Zuschneidbares Wundversorgungsprodukt (9) nach einem der vorhergehenden Ansprüche, wobei die erste proximal gelegene flüssigkeitsdurchlässige Schicht (4) und die distal gelegene Schutzschicht (1) aus einem identischen Material oder einer identischen Materialmischung bestehen.

## Claims

1. Trimmable wound care product (9) containing
a) a proximal liquid-permeable layer (4) comprising a nonwoven,
b) a distal protective layer (1) comprising a nonwoven,
c) a swelling layer (3) located between the proximal liquid-permeable layer (4) and the distal protective layer (1) in the form of a nonwoven comprising superabsorbent, polymer-containing fibres, the superabsorbent fibres being present in a felt with at least one other fibre type,
d) an aqueous saline solution which is stored in the swelling layer (3), which has a pH < 7.0 during storage and which can be delivered to a wound during a wound treatment,
wherein the proximal liquid-permeable layer (4), the swelling layer (3) and the distal protective layer (1) are identically trimmed and lie against each other full face-to-full face without any overhang, and wherein the wound care product (9) is seamless.

2. Trimmable wound care product (9) according to Claim 1, wherein the wound care product (9) has a pressure resistance to a pressure of at least 8 kg / 100 cm², such that the action of such a pressure merely causes elastic deformation of the wound care product (9).

3. Trimmable wound care product (9) according to Claim 1 or 2, wherein the other fibre type is selected from the group consisting of: cellulose-containing fibre, polyester-containing fibre, preferably polyethylene terephthalate, and/or polyamide and wherein the other fibre type is needled with the superabsorbent, polymer-containing fibres, such that it is present in a needle felt.

4. Trimmable wound care product (9) according to any of the preceding claims, wherein the nonwoven of the proximal liquid-permeable layer (4) and/or of the distal protective layer (1) contains thermally consolidated fibres.

5. Trimmable wound care product (9) according to the preceding Claim 4, wherein the thermally consolidated fibres contain or consist of one or more polymers selected from polypropylene, polyvinyl chloride, polyethylene, polyethylene terephthalate, polycarbonate, polyamide, polyurethane, polystyrene, and mixtures thereof.

6. Trimmable wound care product (9) according to any of the preceding claims, comprising additionally a backing layer (5) distal to the protective layer (1) and preferably containing polyurethane.

7. Trimmable wound care product (9) according to Claim 6, wherein the backing layer (5) is elastic, printable and liquid-impermeable and wherein the MVTR is below 1000 g/m²/24 h.

8. Trimmable wound care product (9) according to any of the preceding claims, wherein the superabsorbent fibres in the swelling layer (3) contain a polymer, preferably an acrylate-containing polymer or an acrylate copolymer, which is crosslinkable, the crosslinkability being preferably based on the formation of ester bonds.

9. Trimmable wound care product according to any of the preceding claims, wherein the proximal liquid-permeable layer (4), the swelling layer (3) and the distal protective layer (1) are in the form of a laminate.

10. Trimmable wound care product (9) according to any of the preceding claims, wherein the proximal liquid-permeable layer (4), the swelling layer (3) and the distal protective layer (1) are inseparably bonded to each other face-to-face, the layers being preferably joined together by means of a hot-melt adhesive and the hot-melt adhesive preferably containing or consisting of a polyolefin or copolymer.

11. Trimmable wound care product (9) according to any of the preceding claims, wherein the structural integrity of the wound care product (9) is maintained on trimming, such that the felt structure of the swelling layer does not release fibrous or particulate constituents of the wound care product (9), in particular superabsorbent fibres.

12. Trimmable wound care product (9) according to any of the preceding claims, wherein the wound care product (9) has an additional silicone-containing wound contact layer (7) which is attached, directly or in the form of a coated sheet (6), to the outer face of the liquid-permeable layer (4).

13. Trimmable wound care product (9) according to Claim 12, wherein the silicone-containing wound contact layer (7) only partly overlies the liquid-permeable layer (4) and is preferably perforated.

14. Trimmable wound care product (9) according to any of the preceding claims, wherein the nonwoven of the first proximal liquid-permeable layer (4) and/or of the protective layer (1) in the dry state has a basis weight of 15 g/m² to 50 g/m².

15. Trimmable wound care product (9) according to any of the preceding claims, wherein the first proximal liquid-permeable layer (4) and the distal protective layer (1) consist of an identical material or an identical material blend.

## Revendications

1. Produit de soin de plaies (9) pouvant être découpé à la bonne dimension, contenant
a) une couche perméable aux liquides (4) placée côté proximal et présentant un non-tissé,
b) une couche de protection (1) placée côté distal et présentant un non-tissé,
c) une couche de gonflement (3) placée entre la couche perméable aux liquides (4) placée côté proximal et la couche de protection (1) placée côté distal sous la forme d'un non-tissé présentant des fibres superabsorbantes contenant des polymères, les fibres superabsorbantes se trouvant avec au moins un autre type de fibres dans un feutre,
d) une solution saline aqueuse, qui est stockée dans la couche de gonflement (3), qui présente un pH < 7,0 lorsqu'elle est incorporée et qui peut être administrée à une plaie pendant un traitement de la plaie ;
la couche perméable aux liquides (4) placée côté proximal, la couche de gonflement (3) et la couche de protection (1) placée côté distal présentant une découpe identique et reposant totalement les unes sur les autres sans dépassement et le produit de soin de plaies (9) étant conçu sans couture.

2. Produit de soin de plaies (9) pouvant être découpé à la bonne dimension selon la revendication 1, le produit de soin de plaies (9) présentant une résistance à la pression par rapport à une pression d'au moins 8 kg/100 cm², de telle sorte que le produit de soin de plaies (9) est déformé seulement élastiquement par l'action d'une telle pression.

3. Produit de soin de plaies (9) pouvant être découpé à la bonne dimension selon la revendication 1 ou 2, l'autre type de fibres étant choisi dans le groupe constitué par : les fibres contenant de la cellulose, les fibres contenant du polyester, de préférence le poly(téréphtalate d'éthylène) et/ou le polyamide, et l'autre type de fibres étant aiguilleté avec les fibres superabsorbantes contenant un polymère, de telle sorte qu'il est présent dans un feutre aiguilleté.

4. Produit de soin de plaies (9) pouvant être découpé à la bonne dimension selon l'une des revendications précédentes, le non-tissé de la couche perméable aux liquides (4) placée côté proximal et/ou de la couche de protection (1) placée côté distal contenant des fibres thermiquement solidifiées.

5. Produit de soin de plaies (9) pouvant être découpé à la bonne dimension selon la revendication précédente 4, les fibres thermiquement solidifiées contenant ou étant constituées par un ou plusieurs polymères choisis parmi le polypropylène, le poly(chlorure de vinyle), le polyéthylène, le poly(téréphtalate d'éthylène), le polycarbonate, le polyamide, le polyuréthane, le polystyrène ainsi que les mélanges de ceux-ci.

6. Produit de soin de plaies (9) pouvant être découpé à la bonne dimension selon l'une des revendications précédentes, comprenant en outre une couche arrière (5) placée côté distal par rapport à la couche de protection (1), qui contient de préférence du polyuréthane.

7. Produit de soin de plaies (9) pouvant être découpé à la bonne dimension selon la revendication 6, la couche arrière (5) étant élastique, imprimable et imperméable aux liquides et le MVTR ("moisture vapour transmission rate", taux de perméabilité à la vapeur d'eau) étant inférieur à 1000 g/m²/24h.

8. Produit de soin de plaies (9) pouvant être découpé à la bonne dimension selon l'une des revendications précédentes, les fibres superabsorbantes dans la couche de gonflement (3) contenant un polymère, de préférence un polymère contenant un acrylate ou un copolymère d'acrylate, qui peut être réticulé, l'aptitude à la réticulation reposant de préférence sur la formation de composés de type ester.

9. Produit de soin de plaies pouvant être découpé à la bonne dimension selon l'une des revendications précédentes, la couche perméable aux liquides (4) placée côté proximal, la couche de gonflement (3) et la couche de protection (1) placée côté distal se trouvant sous forme de stratifié.

10. Produit de soin de plaies (9) pouvant être découpé à la bonne dimension selon l'une des revendications précédentes, la couche perméable aux liquides (4) placée côté proximal, la couche de gonflement (3) et la couche de protection (1) placée côté distal étant reliées les unes aux autres à plat et de manière indissociable, les couches étant de préférence réunies entre elles par un adhésif thermofusible et l'adhésif thermofusible contenant ou étant constitué par de préférence une polyoléfine ou un copolymère.

11. Produit de soin de plaies (9) pouvant être découpé à la bonne dimension selon l'une des revendications précédentes, l'intégrité structurale du produit de soin de plaies (9) étant préservée lors de la découpe à la bonne dimension, de telle sorte qu'aucun composant fibreux ou particulaire du produit de soin de plaies (9), en particulier aucune fibre superabsorbante, n'est libéré par la structure de feutre de la couche de gonflement.

12. Produit de soin de plaies (9) pouvant être découpé à la bonne dimension selon l'une des revendications précédentes, le produit de soin de plaies (9) disposant d'une couche supplémentaire de contact avec la plaie (7) contenant de la silicone, qui est appliquée directement ou sous forme d'une feuille (6) revêtue sur la face externe de la couche perméable aux liquides (4).

13. Produit de soin de plaies (9) pouvant être découpé à la bonne dimension selon la revendication 12, la couche de contact avec la plaie (7) contenant de la silicone recouvrant seulement partiellement la couche perméable aux liquides (4) et étant de préférence perforée.

14. Produit de soin de plaies (9) pouvant être découpé à la bonne dimension selon l'une des revendications précédentes, le non-tissé de la première couche perméable aux liquides (4) placée côté proximal et/ou de la couche de protection (1) présentant à l'état sec un poids surfacique de 15 g/m² à 50 g/m².

15. Produit de soin de plaies (9) pouvant être découpé à la bonne dimension selon l'une des revendications précédentes, la première couche perméable aux liquides (4) placée côté proximal et la couche de protection (1) placée côté distal étant constituées d'un matériau identique ou d'un mélange de matériaux identique.
